# FASCICULE DE BREVET EUROPEEN

(11) **EP 1 685 825 B1**
(45) Date de publication et mention de la délivrance du brevet: **24.07.2013**
(21) Numéro de dépôt: 06300077.2
(22) Date de dépôt: 27.01.2006
(51) Int. Cl.: A61Q 5/04, A61K 8/37, A61K 8/87, A61K 8/895, A61K 8/92

(54) **Procédé de traitement capillaire et utilisation dudit procédé**
Haarbehandlungsverfahren und dessen Einsatz
Hair treating process and its uses

(30) Priorité: 28.01.2005 FR 0550249
(43) Date de publication de la demande: 02.08.2006
(73) Titulaire: L'Oréal, 75008 Paris (FR)
(72) Inventeur: Mathonneau, Estelle, 75010, PARIS (FR); Rollat-Corvol, Isabelle, 75017 Paris (FR); Samain, Henri, 91570 Bievres (FR)
(74) Mandataire: Caillet, Isabelle

(56) Documents cités:
- EP-A- 1 064 921
- EP-A- 1 433 467
- EP-A- 1 468 667
- EP-A- 1 475 074
- EP-A- 1 475 076
- EP-A- 1 486 196
- US-A- 4 103 145
- US-A- 5 899 213
- US-A- 5 957 140
- US-A- 5 983 903

## Description

La présente invention est relative à un procédé de traitement capillaire consistant en l'application successive d'un réducteur, d'un rinçage, d'un polymère et/ou d'un stéride, d'une source de chaleur et d'un fixateur, ainsi qu'à l'utilisation dudit procédé.

Les produits de coiffage permettant de donner à la coiffure des formes particulières, originales sont généralement des gels, des solutions ou des mousses contenant un polymère fixant. L'inconvénient lié à ce type de produit réside dans le fait que la tenue de la coiffure est limitée dans le temps : la forme part au premier shampooing.

On connaît par ailleurs des traitements permanents des fibres kératiniques. Ces traitements consistent à réaliser dans un premier temps l'ouverture des liaisons disulfures de la kératine (cystine) à l'aide d'une composition contenant un agent réducteur, puis après avoir de préférence rincé les cheveux, à reconstituer dans un second temps lesdites liaisons disulfures en appliquant sur les cheveux lissés ou préalablement mis sous tension par des moyens adéquats tels que les bigoudis, une composition oxydante encore appelée fixateur de façon à donner à la chevelure la forme recherchée. Cette technique permet indifféremment de réaliser soit l'ondulation des cheveux, soit leur défrisage, leur crêpage ou leur lissage.

Le document EP1468667 A1 divulgue un procédé capillaire pour le lissage des cheveux avec les étapes de réduction, rinçage, mise en forme à l'aide d'un fer chauffant et oxydation.

Le document EP1486196 A1 divulgue un procédé de déformation permanente des cheveux par utilisation d'une composition réductrice comprenant des polymères, rinçage, utilisation des moyens mechaniques de mise en tension et application d'un agent oxydant/fxateur.

Il existe un besoin permanent pour de nouvelles compositions permettant de fixer le coiffure durablement dans des formes très diverses.

La demanderesse a donc mis au point un procédé de traitement des fibres capillaires comprenant au moins les étapes suivantes :
- application sur les fibres capillaires d'un agent réducteur,
- rinçage des fibres capillaires,
- application sur les fibres capillaires d'une composition cosmétique comprenant au moins un polymère fixant non cristallin ou semi-cristallin et/ou au moins un stéride,
- élévation de la température des fibres capillaires au moyen d'une source de chaleur, à une température allant de 50°C à 280°C,
- application sur les fibres capillaires d'un agent oxydant.

La suite de la description se réfère aux figures annexées qui représentent, respectivement :
Figure 1A à 1K : des branches de différents fers chauffants, vus en coupe,
Figure 2A à 2C : des moules adaptables sur des fers chauffants, vus en coupe ou vus de dessus,
Figure 3A à 3J : des fers composés d'un seul élément chauffant, vus en coupe.

La source de chaleur contribue à donner la forme à la coiffure. Elle peut être appliquée par pression sur la chevelure de manière continue ou discontinue .

La source de chaleur est de préférence un ustensile de type fer à lisser, fer à friser, ou fer à cranter permettant d'imprimer des formes originales aux mèches de cheveux.

Le fer permet d'imprimer à la chevelure tous les types de motifs et de forme, et par exemple, des formes en relief, ou en « creux », telles que des formes circulaires ou en elliptiques, par exemple des points ou des ovales, des formes rectangulaires ou hexagonales, petits carrés, rectangles, hexagones, triangles, des motifs imitant des coiffures tels que des tresses, ou tout autres motifs représentant toute forme d'objet, ou d'image.
Pour « imprimer » la forme ou le motif à la chevelure, on peut presser une pince autour d'une mèche de cheveux sur laquelle le réducteur et la composition cosmétique contenant au moins un polymère non cristallin ou semi-cristallin ont été appliqués.

Ainsi, le fer chauffant peut être constitué d'une pince comprenant deux branches, la section de chaque branche pouvant être de forme quelconque et par exemple de forme rectangulaire, incurvée, triangulaire, ou polyhexagonale.

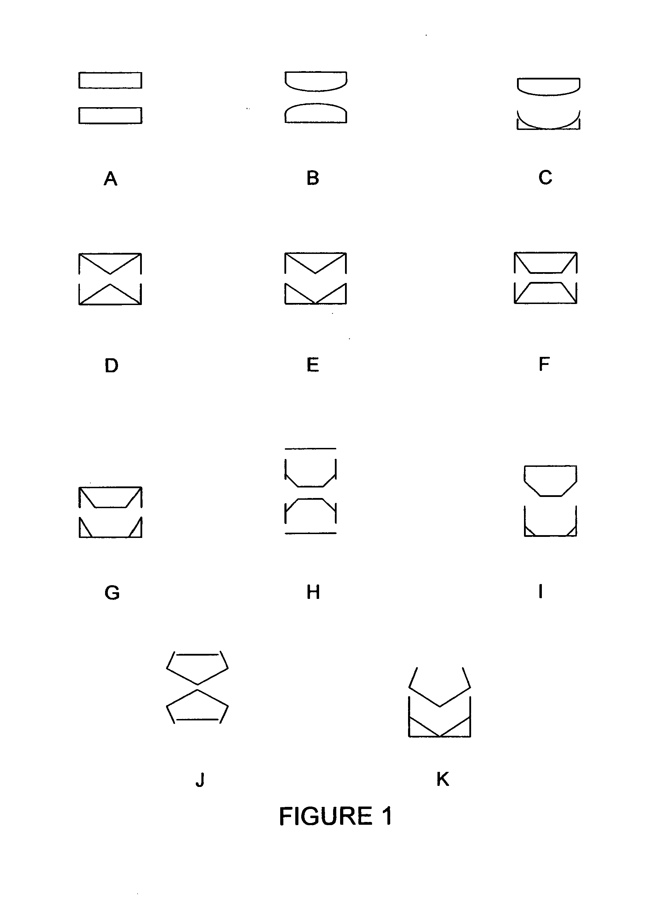

Les branches du fer peuvent posséder une forme symétrique ou complémentaire l'une de l'autre. Une branche ou les deux branches du fer peuvent être chauffantes. Des branches de différents fers chauffants ont été représentés en coupe sur la figure 1.

En particulier, les branches du fer peuvent être plates, incurvées symétriques, incurvées complémentaires, triangulaires symétriques, triangulaires complémentaires, polyhexagonales symétriques, ou polyhexagonales complémentaires.

De manière à imprimer des formes originales aux mèches de cheveux, les branches du fer peuvent être recouvertes par un moule dont la forme peut être très variée.

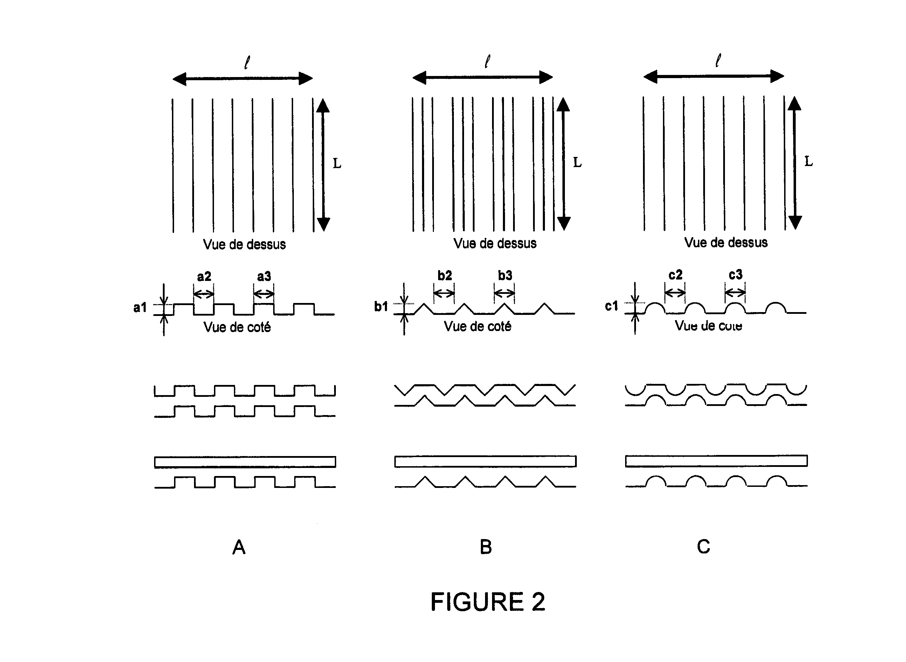

Des exemples de moules pouvant être adaptés à la surface des branches d'un fer sont illustrées sur les figures 2A, 2B et 2C.
Comme cela est représenté sur la figure 2, de préférence, les moules possèdent une largeur « I » variant de 0,5 à 30 cm et de préférence de 2 à 15 cm, une longueur « L » variant de 2 à 30 cm et de préférence, de 5 à 15 cm. En outre les moules, possèderont préférentiellement les dimensions représentées sur les figures 2a, 2b, et 2c, dans lesquelles respectivement,
- a1, b1, c1 varient de 0,1 à 5 cm et de préférence de 0,1 à 1,5 cm,
- a2, b2, c2 varient de 0,1 à 10 cm et de préférence de 0,5 à 5 cm, et
- a3, b3, c3 varient de 0,1 à 10 cm et de préférence de 0,5 à 5 cm.

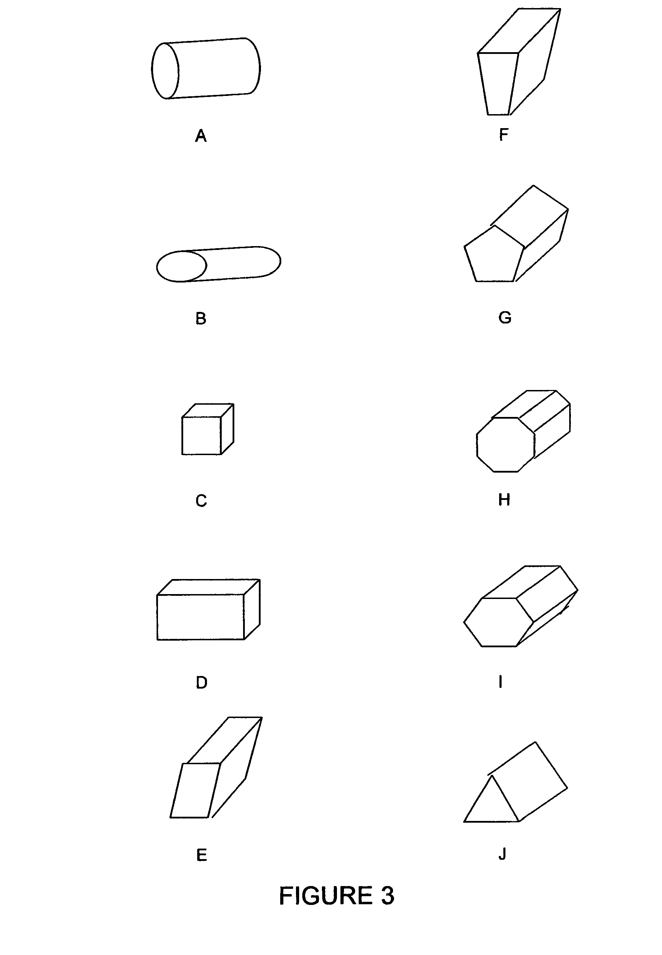

Alternativement, la source de chaleur est un fer composée d'un seul élément chauffant autour duquel les fibres capillaires sont enroulées. Cet élément peut être de forme variée, et par exemple cylindrique ou parallélépipédique. Des exemples de telles sources de chaleur sont illustrées sur la figure 3.

La source de chaleur peut ainsi comprendre un seul élément dont la section a une forme circulaire, ovale, carrée, rectangulaire, parallélépipédique, trapézoïdale, pentagonale, hexagonale, octogonale, ou triangulaire.

Dans un mode de réalisation préféré de l'invention, l'étape d'application sur les fibres capillaires d'une composition cosmétique comprenant au moins un polymère fixant et/ou au moins un stéride et l'étape d'élévation de la température des fibres capillaires sont simultanées.

Le mode de réalisation préféré ci-dessus peut être mis en oeuvre en utilisant un dispositif comprenant une source de chaleur combinée à un réservoir de composition cosmétique contenant le polymère fixant. A titre d'exemple un tel dispositif de conditionnement et d'application peut comprendre :
- Un réservoir, chauffé ou non chauffé,
- Une composition contenant au moins un polymère non cristallin ou semi-cristallin, et/ou au moins un céride, et/ou au moins un stéride,
- Un dispositif d'application,
- Un moyen de chauffage disposé de part et d'autre du dispositif d'application qui permet de chauffer avant, simultanément ou postérieurement à son application la composition cosmétique (température supérieure au point de fusion)
Le moyen de chauffage peut ainsi faire office d'applicateur.

De préférence, l'agent réducteur est choisi parmi les thiols tels que l'acide thioglycolique, l'acide thiolactique, l'acide 3-mercaptopropionique, l'acide thiomalique, l'acide 2,3-dimercaptosuccinique, la cystéine, la N-glycyl-L-cystéine, la L-cystéinylglycine ainsi que leurs esters et sels les thiols tels que l'acide thioglycolique, l'acide thiolactique, l'acide 3-mercaptopropionique, l'acide thiomalique, l'acide 2,3-dimercaptosuccinique, la cystéine, la N-glycyl-L-cystéine, la L-cystéinylglycine ainsi que leurs esters et sels, le thioglycérol, la cystéamine et ses dérivés acylés en C₁-C₄, la N-mésylcystéamine, la N-acétylcystéine, les N-mercaptoalkylamides de sucres tels que le N-(mercapto-2-éthyl) gluconamide, la pantéthéine, les N-(mercaptoalkyl)- ω-hydroxy alkylamides, par exemple ceux décrits dans la demande de brevet EP-A-354 835, les N-mono ou N,N-dialkylmercapto-4 butyramides par exemple ceux décrits dans la demande de brevet EP-A-368 763, les aminomercaptoalkylamides par exemple ceux décrits dans la demande de brevet EP-A-432 000, les dérivés des acides N-(mercaptoalkyl)succinamiques et des N-(mercaptoalkyl)succinimides par exemple ceux décrits dans la demande de brevet EP-A-465 342, les alkylamino mercaptoalkylamides par exemple ceux décrits dans la demande de brevet EP-A-514 282, le mélange azéotrope de thioglycolate de 2-hydroxypropyle et de thioglycolate de (2-hydroxy-1-méthyl)éthyle tel que décrit dans la demande de brevet FR-A-2 679 448, les mercaptoalkylaminoamides par exemple ceux décrits dans la demande de brevet FR-A-2 692 481, les N-mercapto alkyl alcane diamides par exemple ceux décrits dans la demande de brevet EP-A-653 202.

L'agent réducteur, peut alternativement être choisi parmi les hydrures tels que le borohydrure de sodium ou de potassium ou les sulfites ou bisulfites d'un métal alcalin ou alcalino-terreux ; ou encore parmi les dérivés du phosphore tels que les phosphines ou les phosphites.

Les agents réducteurs préférés sont l'acide thioglycolique et la cystéine
ou leurs dérivés. L'agent réducteur est de préférence utilisé en solution aqueuse.

Lorsqu'on utilise un thiol tel que par exemple l'acide thioglycolique, sa concentration est généralement comprise entre 0,05 et 1 M, le pH de la solution aqueuse est de préférence compris entre 6,5 et 9, le temps de contact est généralement compris entre 1 et 15 minutes et de préférence entre 5 et 10 minutes.

Lorsqu'on utilise une cystéine, sa concentration est généralement comprise entre 0.01 et 9%, le pH de la solution aqueuse est de préférence compris entre 6,5 et 10, le temps de contact est généralement compris entre 1 et 15 minutes et de préférence entre 5 et 10 minutes.

Le pH étant ajusté à l'aide d'un agent choisi par exemple parmi :
l'ammoniaque, la monoéthanolamine, la diéthanolamine, la triéthanolamine, la 1,3-propanediamine, un carbonate ou bicarbonate alcalin ou d'ammonium, un carbonate organique tel que le carbonate de guanidine, un hydroxyde alcalin ou encore avantageusement à l'aide d'un hydroxyde d'ammonium quaternaire.

De préférence, l'agent oxydant est choisi parmi le peroxyde d'hydrogène, l'eau oxygénée, le peroxyde d'urée, les bromates de métaux alcalins, les persels, de préférence les perborates et les persulfates, les enzymes, de préférence les peroxydases et les oxydo-réductases à deux électrons.

Par polymère fixant, on entend au sens de la présente invention, un polymère capable de conférer une forme à une chevelure donnée ou capable de maintenir une chevelure dans une forme donnée.

Par polymère fixant non cristallin ou semi cristallin, on entend au sens de la présente invention, tout polymère ne comportant pas de séquence cristallisable dans leur structure, et donc ne présentant pas de température de changement de phase solide-liquide du premier ordre.

Par polymère fixant non cristallin ou semi-cristallin, on entend au sens de la présente invention les polymères fixants cationiques, anioniques, amphotères et non ioniques.

Les polymères fixants cationiques utilisables selon la présente invention sont de préférence choisis parmi les polymères comportant des groupements amine primaire, secondaire, tertiaire et/ou quaternaire faisant partie de la chaîne polymère ou directement reliés à celle-ci, et ayant un poids moléculaire compris entre 500 et environ 5 000 000 et de préférence entre 1000 et 3 000 000.

Parmi ces polymères, on peut citer plus particulièrement les polymères cationiques suivants :
(1) les homopolymères ou copolymères dérivés d'esters ou d'amides acryliques ou méthacryliques et comportant au moins un des motifs suivants : 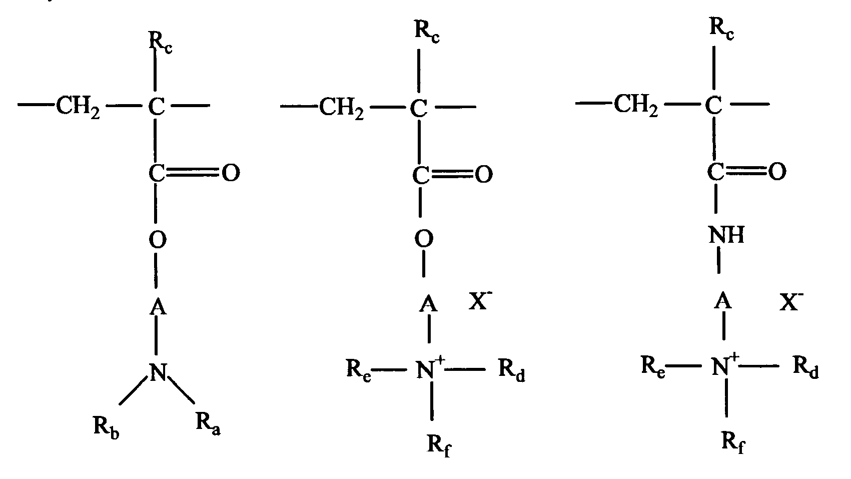 dans lesquels :
   Rₐ et R_{b} représentent chacun un atome d'hydrogène ou un groupe alkyle en C₁₋₆,
   R_{c} désigne un atome d'hydrogène ou un radical CH₃ .
   R_{d}, Rₑ et R_{f}, identiques ou différents, représentent chacun un groupe alkyle en C₁₋₁₈ ou un radical benzyle,
   A est un groupe alkyle linéaire ou ramifié en C₁₋₆ ou un groupe hydroxyalkyle en C₁₋₄, et
   X⁻ désigne un anion méthosulfate ou halogénure tel qu'un ion chlorure ou bromure.

Les copolymères de la famille (1) contiennent en outre un ou plusieurs motifs dérivant de comonomères pouvant être choisis dans la famille des acrylamides, méthacrylamides, diacétone-acrylamides, acrylamides et méthacrylamides substitués sur l'azote par des groupes alkyle inférieurs, des acides acryliques ou méthacryliques ou leurs esters, des vinyllactames tels que la vinylpyrrolidone ou le vinylcaprolactame, et des esters vinyliques.

Ainsi, on peut citer parmi les copolymères de la famille
(1) :
   - les copolymères d'acrylamide et de méthacrylate de diméthylaminoéthyle quaternisés au sulfate de diméthyle ou avec un halogénure de diméthyle, tels que celui vendu sous la dénomination HERCOFLOC® par la Société HERCULES,
   - les copolymères d'acrylamide et de chlorure de méthacryloyloxyéthyltriméthylammonium décrits par exemple dans la demande de brevet EP-A-080976 et vendus sous la dénomination BINA QUAT® P 100 par la Société CIBA GEIGY,
   - le copolymère d'acrylamide et de méthosulfate de méthacryloyloxyéthyltriméthylammonium vendu sous la dénomination RETEN® par la Société HERCULES,
   - les copolymères vinylpyrrolidone/acrylate ou méthacrylate de dialkylaminoalkyle quaternisés ou non, tels que les produits vendus sous la dénomination GAFQUAT® par la Société ISP, comme par exemple GAF-QUAT® 734 OU GAFQUAT® 755, ou bien les produits dénommés COPOLYMER® 845 , 958 et 937. Ces polymères sont décrits en détail dans les demandes de brevets français FR2.077.143 et FR2.393.573,
   - les terpolymères méthacrylate de diméthylaminoéthyle/vinylcaprolactame/vinylpyrrolidone tels que le produit commercialisé sous la dénomination GAFFIX VC 713 par la Société ISP, et
   - le copolymère vinylpyrrolidone/méthacrylamide de diméthylaminopropyle quaternisé tel que le produit vendu sous la dénomination GAFQUAT® HS 100 par la Société ISP.
(2) les polysaccharides quaternisés décrits plus particulièrement par les brevets américains 3.589.578 et 4.031.307 tels que les gommes de guar contenant des groupements cationiques trialkylammonium.
   De tels produits sont commercialisés notamment sous les dénominations commerciales de JAGUAR® C13 S, JAGUAR® C15 et JAGUAR® C17 par la Société MEYHALL.
(3) Les copolymères quaternaires de vinylpyrrolidone et de vinylimidazole tels que les produits commercialisés par BASF sous la dénomination LUVIQUAT® TFC,
(4) les chitosanes ou leurs sels, en particulier les acétate, lactate, glutamate, gluconate ou pyrrolidonecarboxylate de chitosane.
   On peut citer le chitosane ayant un taux de désacétylation de 90,5% en poids vendu sous la dénomination KYTAN BRUT STANDARD® par la Société ABER TECHNOLOGIES, le pyrrolidonecarboxylate de chitosane vendu sous la dénomination KYTAMER® PC par la Société AMERCHOL.
(5) Les dérivés de cellulose cationiques tels que les copolymères de cellulose ou les dérivés de cellulose greffés avec un monomère hydrosoluble comportant un groupe ammonium quaternaire et décrits notamment dans le brevet US 4 131 576 tels que les hydroxyalkylcelluloses, comme les hydroxyméthylhydroxyéthyl- ou hydroxypropylcelluloses greffées notamment avec un sel de méthacryloyloxyéthyltriméthylammonium, de méthacrylamidopropyltriméthylammonium ou de diméthyldiallylammonium.

Les produits commercialisés répondant à cette définition sont plus particulièrement les produits vendus sous les dénominations CELQUAT® L 200 et CELQUAT® H 100 par la Société NATIONAL STARCH.

Les polymères fixants anioniques généralement utilisés sont des polymères comportant des groupements dérivés d'un acide carboxylique, sulfonique ou phosphorique et ont un poids moléculaire moyen en poids compris entre environ 500 et 5 000 000.

Les groupements acide carboxyliques sont apportés par des monomères insaturés contenant une ou deux fonctions acide carboxylique, tels que ceux répondant à la formule : 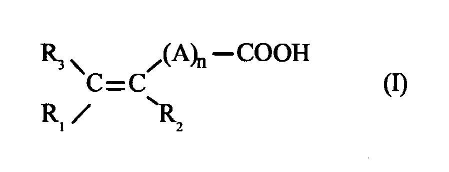 dans laquelle n est un nombre entier de 0 à 10, A désigne un groupement méthylène, éventuellement relié à l'atome de carbone du groupement insaturé
ou du groupement méthylène voisin, lorsque n est supérieur à 1, par l'intermédiaire d'un hétéroatome tel que oxygène ou soufre, R₃ désigne un atome d'hydrogène, un groupement phényle ou benzyle, R₁ désigne un atome d'hydrogène, un groupement alkyle inférieur ou carboxyle, et R₂ désigne un atome d'hydrogène, un groupement alkyle inférieur, un groupement -CH₂₋COOH, phényle ou benzyle.

Dans la formule précitée un radical alkyle inférieur désigne de préférence un groupement ayant de 1 à 4 atomes de carbone et en particulier un groupe méthyle ou éthyle.

Les polymères fixants anioniques carboxylés préférés selon l'invention sont :
A) les homo- ou copolymères d'acide acrylique ou méthacrylique ou leurs sels et en particulier les produits commercialisés sous les dénominations VERSICOL® E ou K par la Société ALLIED COLLOID, et sous la dénomination ULTRAHOLD® par la Société BASF ; les copolymères d'acide acrylique et d'acrylamide commercialisés sous forme de sel de sodium sous les dénominations RETEN® 421, 423 ou 425 par la Société HERCULES ; les sels de sodium des acides polyhydroxycarboxyliques.
B) Les copolymères d'acide acrylique ou d'acide méthacrylique et d'un monomère monoéthylénique tel que l'éthylène, le styrène, les esters vinyliques, les esters d'acide acrylique ou méthacrylique.
   Ces copolymères peuvent être greffés sur un polyalkylène glycol tel que le polyéthylèneglycol et sont éventuellement réticulés.
   De tels polymères sont décrits en particulier dans la demande de brevet français FR 1 222 944 et dans la demande de brevet allemand DE 2 330 956. On peut notamment citer les copolymères comportant dans leur chaîne un motif acrylamide éventuellement N-alkylé et/ou hydroxyalkylé, tels que ceux décrits dans les demandes de brevet luxembourgeois LU 75370 et LU75371 ou proposés sous la dénomination QUADRAMER® par la Société AMERICAN CYANAMID.
   On peut également citer les copolymères d'acide acrylique et de méthacrylate d'alkyle en C₁-C₄, et les terpolymères de vinylpyrrolidone, d'acide (méth)acrylique et de (méth)acrylate d'alkyle en C₁-C₂₀, par exemple de lauryle (ACRYLDONE® LM de la Société ISP), de tertiobutyle (LUVIFLEX® VBM 70 commercialisé par BASF) ou de méthyle (STEPANHOLD® EXTRA commercialisé par STEPAN), et les terpolymères acide méthacrylique/acrylate d'éthyle/acrylate de tertiobutyle tel que le produit commercialisé sous la dénomination LUVIMER® 100 P par la Société BASF.
C) Les copolymères dérivés d'acide crotonique tels que ceux comportant dans leur chaîne des motifs acétate ou propionate de vinyle et éventuellement d'autres monomères tels que les esters allylique, méthallylique ou vinylique d'un acide carboxylique saturé, linéaire ou ramifié, à longue chaîne hydrocarbonée comportant au moins 5 atomes de carbone, ces polymères pouvant éventuellement être greffés et réticulés, ou encore les esters vinylique, allylique ou méthallylique d'un acide carboxylique α- ou β -cyclique.
   De tels polymères sont décrits, entre autres, dans les demandes de brevets français FR 1 222 944, FR 1 580 545, FR2 265 782, FR 2 265 781, FR 1 564 110 et FR 2 439 798.
   On peut citer à titre d'exemples de produits commerciaux entrant dans cette classe les résines 28-29-30, 26-13-14 et 28-13-10 commercialisées par la Société NATIONAL STARCH.
D) Les copolymères dérivés d'acides ou d'anhydrides carboxyliques monoinsaturés en C₄-C₈ choisis parmi :
   - les copolymères comprenant :
      (ii) un ou plusieurs acides ou anhydrides maléique, fumarique, itaconique et
      (iii) au moins un monomère choisi parmi les esters vinyliques, les éthers vinyliques, les halogénures vinyliques, les dérivés phénylvinyliques, l'acide acrylique et ses esters, les fonctions anhydride de ces copolymères étant éventuellement monoestérifiées ou monoamidifiées.

De tels polymères sont décrits en particulier dans les demandes de brevets US 2,047,398, US 2,723,248, US 2,102,113 et GB 839,805 et notamment ceux commercialisés sous les dénominations GANTREZ AN ou ES, AVANTAGE® CP par la Société ISP ;
- les copolymères comprenant (i) un ou plusieurs anhydrides maléique, citraconique ou itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne, les fonctions anhydrides de ces copolymères étant éventuellement monoestérifiées ou monoamidifiées.

Ces polymères sont par exemple décrits dans les demandes de brevets français FR 2 350 384 et FR 2 357 241 de la demanderesse.
E) Les polyacrylamides comportant des groupements carboxylate.

Les groupements anioniques des polymères fixants anioniques de la présente invention peuvent également être des groupes acide sulfonique apportés par des motifs vinylsulfonique, styrènesulfonique, naphtalènesulfonique ou acrylamidoalkylsulfonique.

Ces polymères à groupes acide sulfonique sont notamment choisis parmi :
- les sels de poly(acide vinylsulfonique) ayant un poids moléculaire moyen en poids compris entre environ 1000 et 100 000 ainsi que les copolymères d'acide vinylsulfonique et d'un comonomère insaturé tel que l'acide acrylique, l'acide méthacrylique, les esters de ces acides, l'acrylamide, les dérivés d'acrylamide, les éthers vinyliques et la vinylpyrrolidone ;
- les sels de poly(acide styrènesulfonique). On peut citer à titre d'exemple deux sels de sodium ayant un poids moléculaire moyen en poids d'environ 500 000 et d'environ 100 000 commercialisés respectivement sous les dénominations FLEXAN® 500 et FLEXAN® 130 par la Société NATIONAL STARCH. Ces composés sont décrits dans le brevet FR 2 198 719 ;
- les sels de poly(acide acrylamidesulfonique), tels que ceux mentionnés dans le brevet US 4,128,631 et plus particulièrement le poly(acide acrylamidoéthyl-propanesulfonique) commercialisé sous la dénomination COSMEDIA POLYMER® HSP 1180 par la Société HENKEL.

Selon l'invention, les polymères fixants anioniques sont de préférence choisis parmi les copolymères d'acide acrylique tels que les terpolymères acide acrylique/acrylate d'éthyle/N-tertiobutylacrylamide commercialisés notamment sous la dénomination ULTRAHOLD STRONG® par la Société BASF, les copolymères dérivés d'acide crotonique tels que les terpolymères acétate de vinyle/tertio-butyl benzoate de vinyle/acide crotonique et les terpolymères acide crotonique/acétate de vinyle/néododécanoate de vinyle commercialisés notamment sous la dénomination Résine 28-29-30 par la Société NATIONAL STARCH, les copolymères dérivés d'acide ou d'anhydride maléique, fumarique ou itaconique et contenant comme comonomères des esters vinyliques, des éthers vinyliques, des halogénures vinyliques, des dérivés phénylvinyliques, l'acide acrylique et les esters d'acide acrylique, tels que les copolymères méthylvinyléther/anhydride maléique monoestérifié, commercialisés par exemple sous la dénomination GANTREZ® par la Société ISP, les copolymères d'acide méthacrylate de méthyle commercialisés sous la dénomination EUDRAGIT®L par la Société ROHM PHARMA, les copolymères acide méthacrylique/méthacrylate de méthyle/acrylate d'alkyle en C₁₋₄/acide acrylique ou méthacrylate d'hydroxyalkyle en C₁₋₄, commercialisés sous la dénomination AMERHOLD® DR 25 par la Société AMERCHOL, ou sous la dénomination ACUDYNE® 255 par la Société ROHM & HAAS, les copolymères d'acide méthacrylique et d'acrylate d'éthyle commercialisés sous la dénomination LUVIMER® MAEX ou MAE par la Société BASF et les copolymères acétate de vinyle/acide crotonique et les copolymères acétate de vinyle/acide crotonique greffés par du polyéthylèneglycol commercialisés sous la dénomination ARISTOFLEX® A par la Société BASF.

Les polymères fixants amphotères utilisables pour la présente invention sont choisis notamment parmi les polymères comportant des motifs B et C répartis statistiquement dans la chaîne polymère, où B désigne un motif dérivant d'un monomère comportant au moins un atome d'azote basique et C désigne un motif dérivant d'un monomère comportant un ou plusieurs groupements acide carboxylique ou acide sulfonique. Les polymères fixants amphotères peuvent également comporter des motifs zwittérioniques de type carboxybétaïne ou sulfobétaïne. Il peut également s'agir de polymères à chaîne principale cationique comportant des groupements amine primaire, secondaire, tertiaire ou quaternaire, parmi lesquels au moins un, porte, par l'intermédiaire d'un radical hydrocarboné, un groupement acide carboxylique ou acide sulfonique. Les polymères fixants amphotères peuvent encore avoir une chaîne anionique dérivée d'acides dicarboxyliques α,β-insaturés dont l'un des groupements carbonyle a été amené à réagir avec une polyamine comportant un ou plusieurs groupements amine primaire ou secondaire.

Les polymères fixants amphotères répondant à la définition donnée ci-dessus sont choisis en particulier parmi les polymères suivants :
(1) Les polymères résultant de la copolymérisation d'un monomère vinylique portant un groupement acide carboxylique tel que l'acide acrylique, l'acide méthacrylique, l'acide maléique, l'acide α -chloroacrylique, et d'un monomère vinylique contenant au moins une fonction basique tel que les méthacrylate et acrylate de dialkylaminoalkyle ou les dialkylaminoalkyl(méth)acrylamides. De tels composés sont décrits par exemple dans la demande de brevet américain US 3,836,537.
(2) Les polymères comportant des motifs dérivés :
   (a) d'au moins un monomère choisi parmi les acrylamides ou méthacrylamides N-alkylés,
   (b) d'au moins un comonomère contenant une ou plusieurs fonctions acide carboxylique, et
   (c) d'au moins un comonomère basique tel que des esters à substituants amine primaire, secondaire, tertiaire ou quaternaire d'acide acrylique et d'acide méthacrylique, et le produit de quaternisation du méthacrylate de diméthylaminoéthyle avec le sulfate de diméthyle ou diéthyle.

Les acrylamides ou méthacrymlamides N-alkylés (a) préférés sont ceux portant des radicaux alkyle en C₂₋₁₂ , tels que le N-éthylacrylamide, le N-tertiobutylacrylamide, le N-tertio-octylacrylamide, le N-octylacrylamide, le N-décylacrylamide, le N-dodécylacrylamide ainsi que les méthacrylamides correspondants.

Les comonomères à groupe acide carboxylique (b) sont choisis plus particulièrement parmi les acides acrylique, méthacrylique, crotonique, itaconique, maléique, fumarique ainsi que parmi les monoesters d'alkyle en C₁₋₄ des acides ou des anhydrides maléique ou fumarique.

Les comonomères basiques (c) préférés sont le méthacrylate d'aminoéthyle, le méthacrylate de butylaminoéthyle, le méthacrylate de N,N'-diméthylaminoéthyle et le méthacrylate de N-tertiobutylaminoéthyle.

On utilise en particulier les copolymères dont la dénomination CTFA (4ième Ed., 1991) est « Octylacrylamide/acrylates/butylaminoéthylméthacrylate copolymer », tels que les produits commercialisés sous la dénomination AMPHOMER® ou LOVOCRYL® 47 par la Société NATIONAL STARCH.
(3) Les polyaminoamides réticulés et alcoylés, dérivés en partie ou en totalité de polyaminoamides de formule générale :

   (II) **-[C(=0)-R₄-C(=0)-Z-]-**

   dans laquelle R₄ représente un radical divalent dérivé d'un acide dicarboxylique saturé, d'un acide aliphatique mono- ou dicarboxylique à double liaison éthylénique, d'un ester d'alkyle en C₁₋₆ de ces acides ou d'un radical dérivant de l'addition de l'un quelconque desdits acides sur une amine bis primaire ou bis secondaire, et Z désigne un radical d'une polyalkylène-polyamine bis-primaire, mono- ou bis-secondaire, et de préférence représente :
   a) dans les proportions de 60 à 100 % en moles, le radical :

      (III) **-NH-[(CH₂)ₓ-NH]ₚ-**

      où x = 1 et p = 2 ou 3, ou bien x = 3 et p = 2,
      ce radical dérivant de la diéthylènetriamine, de la triéthylènetétraamine ou de la dipropylènetriamine ;
   b) dans les proportions de 0 à 40% en moles le radical de formule (III)
      dans lequel x = 2 et p = 1, dérivé de l'éthylènediamine, ou le radical 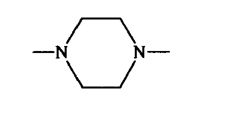 dérivé de la pipérazine :
   c) dans les proportions de 0 à 20 % en moles le radical -NH-(CH₂)₆-NH-dérivé de l'hexaméthylènediamine, ces polyaminoamines étant réticulées par addition de 0,025 à 0,35 mole par mole de groupement amine, d'un agent réticulant bifonctionnel choisi parmi les épihalohydrines, les diépoxydes, les dianhydrides, les composés di-insaturés, et alcoylés par l'acide acrylique, l'acide chloracétique ou une alcane-sulfone.

Les acides carboxyliques saturés sont choisis de préférence parmi les acides ayant 6 à 10 atomes de carbone tels que l'acide adipique, l'acide triméthyl-2,2-4-adipique et triméthyl-2,4,4-adipique, l'acide téréphtalique, les acides à double liaison éthylénique comme par exemple les acides acrylique, méthacrylique, itaconique.

Les alcane-sulfones utilisées dans l'alcoylation sont de préférence la propanesulfone ou la butanesulfone.

Les sels des agents d'alcoylation sont de préférence les sels de sodium
ou de potassium.
(4) Les polymères comportant des motifs zwittérionique de formule : 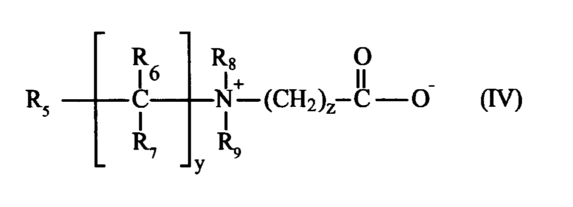 dans laquelle R₅ désigne un groupement insaturé polymérisable tel qu'un groupement acrylate, méthacrylate, acrylamide ou méthacrylamide, y et z représentent chacun un nombre entier de 1 à 3, R₆ et R₇ représentent chacun indépendamment un atome d'hydrogène ou un groupe méthyle, éthyle ou propyle, R₈ et R₉ représentent chacun indépendamment un atome d'hydrogène
   ou un radical alkyle, le nombre total d'atomes de carbone dans R₈ et R₉ ne dépassant pas 10.

Les polymères comprenant de tels motifs de formule (IV) peuvent comporter en outre des motifs dérivés de monomères non zwittérioniques tels que l'acrylate ou le méthacrylate de diméthyle ou de diéthylaminoéthyle, les acrylates ou méthacrylates d'alkyle, les acrylamides ou méthacrylamides ou l'acétate de vinyle.

A titre d'exemple, on peut citer le copolymère méthacrylate de méthyle/méthacrylate de diméthylcarboxyméthylammonioéthyle tel que le produit commercialisé sous la dénomination DIAFORMER® Z301 par la Société SANDOZ.
(5) Les polymères dérivés du chitosane comportant des motifs monomères répondant aux formules suivantes : 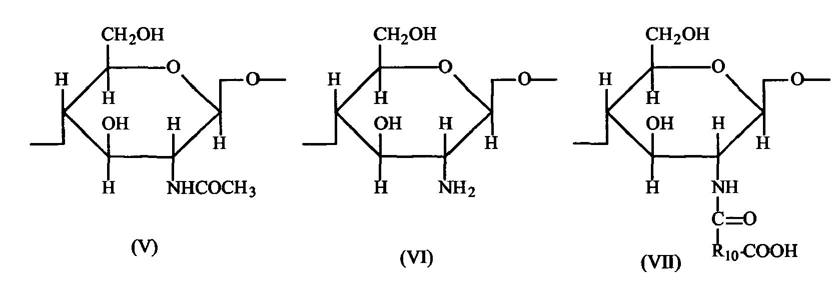 le motif de formule (V) étant présent dans des proportions comprises entre 0 et 30%, le motif de formule (VI) dans des proportions comprises entre 5 et 50% et le motif de formule (VII) dans des proportions comprises entre 30 et 90%, étant entendu que dans ce motif (VII), R₁₀ représente un radical de formule : 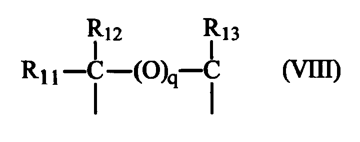 dans laquelle :
   si q = 0, alors R₁₁, R₁₂ et R₁₃, identiques ou différents, représentent chacun un atome d'hydrogène, un groupe méthyle, hydroxyle, acétoxy ou amino, un groupe monoalcoylamine ou dialcoylamine éventuellement interrompu par un ou plusieurs atomes d'azote et/ou éventuellement substitués par un ou plusieurs groupes amine, hydroxyle, carboxyle, alcoylthio ou sulfo, un groupe alcoylthio dont le groupe alcoyle porte un reste amino, l'un au moins des radicaux R₁₁, R₁₂ et R₁₃ étant dans ce cas un atome d'hydrogène ; ou si q = 1, alors R₁₁, R₁₂ et R₁₃ représentent chacun un atome d'hydrogène, ainsi que les sels formés par ces composés avec des bases ou des acides.
6) Les polymères obtenus par N-carboxyalkylation du chitosane, comme le N-carboxyméthylchitosane ou le N-carboxybutylchitosane commercialisé sous la dénomination EVALSAN® par la Société JAN DEKKER.
(7) Les polymères ayant comme unités répétitives celles de formule générale (IX) : 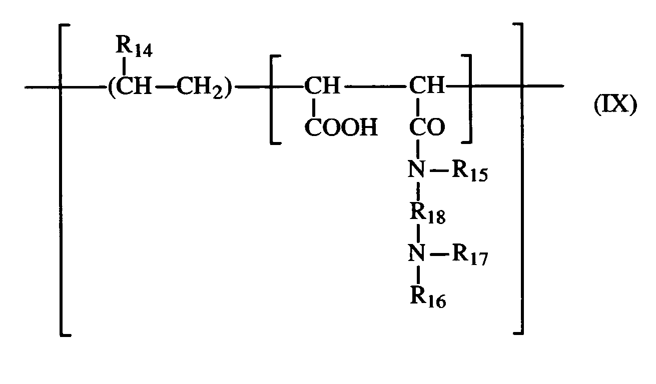 décrits notamment dans la demande de brevet FR 1 400 366, dans laquelle R₁₄ représente un atome d'hydrogène ou un radical CH₃O, CH₃CH₂O ou phényle, R₁₅ désigne un atome d'hydrogène ou un radical alkyle inférieur tel que méthyle ou éthyle, R₁₆ désigne un atome d'hydrogène ou un radical alkyle inférieur tel que méthyle ou éthyle, R₁₇ désigne un radical alkyle inférieur tel que méthyle ou éthyle ou un radical répondant à la formule :

   -R₁₈-N(R₁₆)₂,

   R₁₈ représentant un groupement -CH₂ -CH₂-,-CH₂ -CH₂- CH₂-, -CH2-CH(CH₃)-, et R₁₆ ayant les significations mentionnées ci-dessus, ainsi que les homologues supérieurs de ces radicaux contenant jusqu'à 6 atomes de carbone.
(8) Les polymères amphotères du type -D-X-D-X-choisis parmi :
   (a) les polymères obtenus par action d'acide chloroacétique ou de chloroacétate de sodium sur les composés comportant au moins un motif de formule :

      (X) -D-X-D-X-D-
      où D désigne un radical : 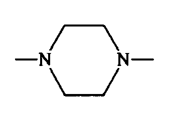 et X désigne le symbole E ou E', E ou E' identiques ou différents désignant un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote ou de soufre, 1 à 3 cycles aromatiques et/ou hétérocycliques, les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule :

      (X') -D-X'-D-X'-

      où D désigne un radical 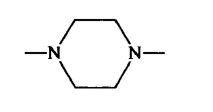 et X' désigne le symbole E ou E' et au moins une fois E', E ayant la signification indiquée ci-dessus et E' étant un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisée par réaction avec l'acide chloroacétique ou du chloroacétate de soude.
9) Les copolymères alkyl(C₁₋₅)vinyléther/anhydride maléique modifiés partiellement par semi-amidification avec une N,N-dialkylaminoalkylamine telle que la N,N-diméthylaminopropylamine, ou par semi-estérification avec une N,N-dialcanolamine. Ces copolymères peuvent également comporter d'autres comonomères vinyliques tels que le vinylcaprolactame.
   Les polymères fixants amphotères préférés selon l'invention sont ceux de la famille (3) décrite ci-dessus, tels que ceux dont la dénomination CTFA est « Octylacrylamide/acrylates/butylaminoéthyl-méthacrylate copolymer ». On peut citer à titre d'exemple les produits commercialisés sous les dénominations AMPHOMER®, AMPHOMER® LV 71 ou LOVOCRYL® 47 par la Société NATIONAL STARCH.

D'autres polymères fixants amphotères préférés sont ceux de la famille (4), comme par exemple les copolymères de méthacrylate de méthyle et de méthacrylate de diméthylcarboxyméthylammonioéthyle, commercialisés par exemple sous la dénomination DIAFORMER® Z301 par la Société SANDOZ.

Les polymères fixants anioniques ou amphotères peuvent, si nécessaire, être partiellement ou totalement neutralisés. Les agents de neutralisation sont par exemple la soude, la potasse, l'amino-2-méthylpropanol, la monoéthanolamine, la triéthanolamine ou la tri-isopropanolamine, les acides minéraux ou organiques tels que l'acide chlorhydrique ou l'acide citrique.

Les polymères fixants non ioniques utilisables selon la présente invention sont choisis par exemple parmi :
- les homopolymères de vinylpyrrolidone,
- les copolymères de vinylpyrrolidone et d'acétate de vinyle,
- les polyalkyloxazolines telles que les polyéthyloxazolines proposées par la Société DOW CHEMICAL sous les dénominations PEOX® 50 000 , PEOX® 200 000 et PEOX® 500 000,
- les homopolymères d'acétate de vinyle tels que le produit proposé sous le nom de APPRETAN® EM par la Société HOECHST ou le produit proposé sous le nom de RHODOPAS® A 012 par la Société RHONE POULENC ;
- les copolymères d'acétate de vinyle et d'esters acryliques tels que le produit proposé sous la dénomination RHODOPAS® AD 310 par RHONE POULENC,
- les copolymères d'acétate de vinyle et d'éthylène tels que le produit proposé sous le nom de APPRETAN® TV par la Société HOECHST,
- les copolymères d'acétate de vinyle et d'ester maléique par exemple de maléate de dibutyle tels que le produit proposé sous le nom de APPRETAN® MB EXTRA par la Société HOECHST,
- les copolymères d'éthylène et d'anhydride maléique,
- les homopolymères d'acrylates d'alkyle et les homopolymères de méthacrylates d'alkyle tels que le produit proposé sous la dénomination MICROPEARL® RQ 750 par la Société MATSUMOTO ou le produit proposé sous la dénomination LUHYDRAN® A 848 S par la Société BASF,
- les copolymères d'esters acryliques tels que par exemple les copolymères d'acrylates d'alkyle et de méthacrylates d'alkyle tels que les produits proposés par la Société ROHM & HAAS sous les dénominations PRIMAL AC-261 K et EUDRAGIT NE 30 D, par la Société BASF sous les dénominations ACRONAL® 601, LUHYDRAN® LR 8833 ou 8845, et par la Société HOECHST sous les dénominations APPRETAN® N 9213 ou N 9212,
- les copolymères d'acrylonitrile et d'un monomère non ionique choisi par exemple parmi le butadiène et les (méth) acrylates d'alkyle ; on peut citer les produits proposés sous les dénominations NIPOL® LX 531 B par la Société NIPPON ZEON
ou ceux proposés sous la dénomination CJ 0610 B par la Société ROHM & HAAS,
- les polyuréthanes tels que les produits proposés sous les dénominations ACRYSOL® RM 1020 OU ACRYSOL® RM 2020 par la Société ROHM & HAAS, les produits URAFLEX® XP 401 UZ, URAFLEX® XP et 402 UZ par la Société DSM RESINS,
- les copolymères d'acrylate d'alkyle et d'uréthanne tels que le produit 8538-33 commercialisé par la Société NATIONAL STARCH,
- les polyamides tels que le produit ESTAPOR® LO 11 proposé par la Société RHONE POULENC,
- les gommes de guar non ioniques chimiquement modifiées ou non modifiées.

Les gommes de guar non ioniques non modifiées sont par exemple les produits vendus sous la dénomination VIDOGUM® GH 175 par la Société UNIPECTINE et sous la dénomination JAGUAR® C par la Société MEYHALL. Les gommes de guar non ioniques modifiées utilisables selon l'invention sont de préférence modifiées par des groupements hydroxyalkyle en C₁₋₈. On peut mentionner à titre d'exemple les groupements hydroxyméthyle, hydroxyéthyle, hydroxypropyle et hydroxybutyle.

Ces gommes de guar sont bien connues dans la technique et peuvent par exemple être préparées par réaction des oxydes d'alcènes correspondants, tels que par exemple des oxydes de propylène, avec la gomme de guar de façon à obtenir une gomme de guar modifiée par des groupements hydroxypropyle.

De telles gommes de guar non ioniques éventuellement modifiées par des groupements hydroxyalkyle sont par exemple vendues sous les dénominations commerciales JAGUAR® HP8, JAGUAR® HP60 et JAGUAR® HP120, JAGUAR® DC 293 et JAGUAR® HP 105 par la Société MEYHALL, ou sous la dénomination GALACTASOL® 4H4FD2 par la Société AQUALON.

Selon l'invention, on peut également utiliser, en tant que polymères fixants, des polymères filmogènes de type silicone greffée comprenant une partie polysiloxane et une partie constituée d'une chaîne organique non siliconée, l'une des deux parties constituant la chaîne principale du polymère et l'autre étant greffée sur ladite chaîne principale.

Ces polymères sont par exemple décrits dans les demandes de brevet EP-A-0 412 704, EP-A-0 412 707, EP-A-0 640 105 et WO 95/00578, EP-A-0582 152 et WO 93/23009 et les demandes de brevets US 4 693 935, US 4 728 571 et US 4 972 037.

Ces polymères sont de préférence anioniques ou non ioniques.

De tels polymères sont par exemple les copolymères susceptibles d'être obtenus par polymérisation radicalaire à partir du mélange de monomère formé,
a) de 50 à 90% en poids d'acrylate de tertiobutyle,
b) de 0 à 40% en poids d'acide acrylique,
c) de 5 à 40% en poids d'un macromère siliconé de formule : 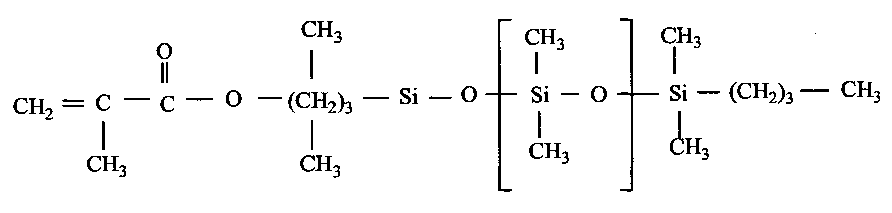 avec v étant un nombre allant de 5 à 700, les pourcentages en poids calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

On peut aussi utiliser comme polymères fixants des polyuréthannes fonctionnalisés ou non, siliconés ou non.

Les polyuréthannes particulièrement visés par la présente invention sont ceux décrits dans les demandes de brevets EP 0 751 162, EP 0 637 600, FR 2 743 297 et EP 0 648 485 de la demanderesse, ainsi que la demande de brevet EP 0 656 021 ou WO 94/03510 de la Société BASF et EP 0 619 111 de la Société NATIONAL STARCH.

Les cérides sont des mono esters d'acides gras et d'alcools aliphatiques à longue chaîne. De préférence, ces alcools aliphatiques sont des alcools primaires à nombre pair de carbones saturés et non ramifiés. La longueur des chaines carbonées varie de 14 à 30 atomes de carbone pour l'acide gras, et de 16 à 36 atomes de carbone pour l'alcool gras. A titre d'exemple de céride on peut citer le palmitate de cétyle ou le palmitate de céryle ou myricyle.

Les cérides sont des constituants importants des cires animales ou végétales. Ces dernières peuvent être utilisées comme source de cérides pour les compositions de l'invention. On peut en particulier citer la cire de carnauba, la cire de candellila, ou la cire d'abeille.

Les stérides résultent de l'estérification d'acides gras par des stérols. Parmi les stérols on peut citer le cholestérol, l'ergostérol, le lanostérol, l'agnostérol, le stigmastérol ou le fucostérol. Les acides gras comportent de 14 à 30 atomes de carbones. A titre de stérides on peut citer le palmitate de cholestéryle. Comme source de stérides, on peut citer la lanoline.

De préférence, l'actif présent dans la composition cosmétique selon l'invention est un polymère fixant.

Dans un mode de réalisation préféré du procédé de l'invention, la composition cosmétique utilisée, comprenant au moins un polymère fixant non cristallin ou semi-cristallin, comprend aussi, un composé choisi parmi les silicones sous forme soluble, dispersée, micro ou nano dispersée, volatiles ou non volatiles, les agents épaississants, les agents tensioactifs non ioniques, anioniques, cationiques et amphotères, les agents conditionneurs, les agents adoucissants, les agents anti-mousse, les agents hydratants, les agents émollients, les plastifiants, les filtres solaires hydrosolubles et liposolubles, siliconés ou non siliconés, les colorants permanents ou temporaires, les pigments minéraux ou organiques, colorés ou non colorés, les charges minérales, les argiles, les nacres ou agents, les opacifiants, les colloïdaux , les parfums, les peptisants, les conservateurs, les céramides, et pseudo-céramides, les vitamines et les provitamines dont le panthénol, les protéines, les agents séquestrants, les agents solubilisants, les agents, les agents alcanisants, les agents anti-corrosion, les corps gras autres que les cérides et les stérides, tels que les huiles végétales, animales, minérales et synthétiques, et leurs mélanges.

De préférence, la composition cosmétique décrite ci-dessus est une composition liquide comprenant un solvant physiologiquement acceptable constitué par de l'eau ou par un ou plusieurs solvants organiques, ou encore par un mélange d'eau et d'un ou plusieurs solvants organiques.

Le solvant organique peut être choisi dans le groupe comprenant l'eau, les alcools inférieurs en C1 à C4 tels que l'éthanol, l'isopropanol, l'acétone, la méthylethylcétone, l'acétate d'éthyle, l'acétate de méthyle, l'acétate de butyle, le diethoxyéthane, le diméthoxyéthane, les alcanes C6 à C10 et leurs mélanges.

La composition cosmétique peut comprendre, par rapport au poids total de la composition, 0,1% à 30% en poids de polymères fixants non cristallin ou semi-cristallin et/ou de cérides, et/ou de stérides, et de préférence 0,5% à 20% en poids de polymères fixant non cristallin ou semi-cristallin et/ou de cérides, et/ou de stérides.

La composition cosmétique peut comprendre, par rapport au poids total de la composition, 0,0001% à 40% en poids d'adjuvant cosmétique, et de préférence 0,001% à 20% en poids d'adjuvant cosmétique tels que décrits ci-dessus.

L'invention concerne également l'utilisation du procédé décrit ci-dessus pour modifier de manière durable la forme de la chevelure.

L'exemple suivant est destiné à illustrer l'invention sans pour autant présenter un caractère limitatif.

### Exemples

### Formules possibles de l'invention

**Composition A**

| | | |
|---|---|---|
| Polyuréthane (1) | 5.0% | MA |
| Parfum | 0.4% | |
| Eau | 94.6% | |

| | | |
|---|---|---|
| (1) « Luviset Si Pur » commercialisé par BASF | | |

**Composition B (hors de l'invention)**

| | |
|---|---|
| Microdispersion de cire de carnauba (MEROXYL SAP commercialisé par CHIMEX) | 26g (soit 7% MA, soit 5,9% de cérides) |
| Eau | qsp100g |

**Composition C**

| | |
|---|---|
| Lanoline | 100 % |

### Procédé d'application :

- 1.: Appliquer une solution d'acide thioglycolique (1 M, pH = 9, pH ajusté avec ammoniaque). On laisse pénétrer d'acide thioglycolique 5 minutes au coeur de la fibre
- 2.: Rincer la mèche de cheveux et pré-sèche éventuellement
- 3.: Appliquer la composition cosmétique A
- 4.: Appliquer un fer à cranter pour former des crans, ou un fer à friser pour former des boucles ou tout autre sorte de fer pour donner une forme,
- 5.: Appliquer ensuite une solution d'eau oxygénée à 8 vol pH 3 (pH ajusté avec l'acide citrique), on laisse pose 5 minutes
- 6.: Rincer la chevelure et éliminer la composition cosmétique ayant permis de maintenir la forme avec un shampooing ou un solvant.

On obtient une chevelure aux formes durables : des crans, des boucles, des ondulations suivant les fers utilisés. On obtient le même type de résultat en utilisant les compositions B et C à la place de la composition A.

## Revendications

1. Procédé de traitement des fibres capillaires permettant d'imprimer à la chevelure tout types de motifs et de formes **caractérisé en ce qu'**il comprend au moins les étapes successives suivantes :
- application sur les fibres capillaires d'un agent réducteur,
- rinçage des fibres capillaires,
- application sur les fibres capillaires d'une composition cosmétique comprenant au moins un stéride et/ou au moins un polymère fixant non cristallin ou semi-cristallin,
- élévation de la température des fibres capillaires au moyen d'une source de chaleur, à une température allant de 50°C à 280°C, ladite source de chaleur étant un fer à friser, un fer à lisser ou un fer à cranter.
- application sur les fibres capillaires d'un agent oxydant.

2. Procédé de traitement selon la revendication 1, **caractérisé en ce que** le polymère fixant non cristallin ou semi-cristallin est choisi parmi les polymères fixant anioniques, cationiques, amphotères ou non ioniques.

3. Procédé de traitement selon l'une quelconque des revendications 1 et 2, **caractérisé en ce que** le fer est constitué d'une pince comprenant deux branches, la section de chaque branche étant de forme rectangulaire, incurvée, triangulaire, ou polyhexagonale.

4. Procédé de traitement selon la revendication 3, **caractérisé en ce que** les branches de la pince possèdent une section symétrique ou complémentaire l'une de l'autre.

5. Procédé de traitement selon l'une quelconque des revendications 1 et 2, **caractérisé en ce que** le fer est composé d'un seul élément chauffant autour duquel les fibres capillaires sont enroulées.

6. Procédé de traitement selon la revendication 5, **caractérisé en ce que** ledit élément possède une section dont la forme est choisie parmi une forme circulaire, ovale, carrée, rectangulaire, parallélépipédique, trapézoïdale, pentagonale, hexagonale, octogonale, et triangulaire.

7. Procédé de traitement selon l'une quelconque des revendications 1 à 6, **caractérisé en ce que** l'étape d'application sur les fibres capillaires d'une composition cosmétique comprenant au moins un polymère fixant non cristallin ou semi-cristallin, et/ou au moins un stéride et l'étape d'élévation de la température des fibres capillaires sont simultanées.

8. Procédé de traitement selon l'une quelconque des revendications 1 à 7, **caractérisé en ce que** l'agent réducteur est choisi parmi les thiols tels que l'acide thioglycolique, l'acide thiolactique, l'acide 3-mercaptopropionique, l'acide thiomalique, l'acide 2,3-dimercaptosuccinique, la cystéine, la N-glycyl-L-cystéine, la L-cystéinylglycine ainsi que leurs esters et sels les thiols tels que l'acide thioglycolique, l'acide thiolactique, l'acide 3-mercaptopropioniqua, l'acide thiomalique, l'acide 2,3-dimercaptosuccinique, la cystéine, la N-glycyl-L-cystéine, la L-cystéinylglycine ainsi que leurs esters et sels, le thioglycérol, la cystéamine et ses dérivés acylés en C₁-C₄, la N-mésylcystéamine, la N-acétylcystéine, les N-mercaptoalkylamides de sucres tels que le N-(mercapto-2-éthyl) gluconamide, la pantéthéine, les N-(mercaptoalkyl)- ω-hydroxy alkylamides, les N-mono ou N,N-dialkylmercapto-4 butyramides, les aminomercaptoalkylamides, les dérivés des acides N-(mercapto-alkyl)succinamiques et des N-(mercaptoalkyl)succinimides, les alkylamino mercaptoalkylamides, le mélange azéotrope de thioglycolate de 2-hydroxypropyle et de thioglycolate de (2-hydroxy-1-méthyl)éthyle, les mercaptoalkylamineamides, les N-mercapto alkyl alcane diamides, et leurs mélanges.

9. Procédé de traitement selon l'une quelconque des revendications 1 à 8, **caractérisé en ce que** l'agent oxydant est choisi parmi le peroxyde d'hydrogène, l'eau oxygénée, le peroxyde d'urée, les bromates de métaux alcalins, les persels, de préférence les perborates et les persulfates, les enzymes, de préférence les peroxydases et les oxydo-réductases à deux électrons.

10. Procédé de traitement selon l'une quelconque des revendications 1 à 9, **caractérisé en ce que** la composition cosmétique comprend en outre au moins un céride

11. Procédé selon la revendication 10, ledit céride étant un mono ester d'acides gras en C14 à C30 et d'alcools primaires saturés et non ramifiés en C16 à C36.

12. Procédé de traitement selon l'une quelconque des revendications 1 à 11, **caractérisé en ce que** le céride est obtenu à partir de cires de carnauba, de candelilla ou d'abeille.

13. Procédé de traitement selon l'une quelconque des revendications 1 à 12, **caractérisé en ce que** le stéride est un ester d'acides gras en C14 à C30 et de stérols.

14. Procédé de traitement selon l'une quelconque des revendications 1 à 13, **caractérisé en ce que** le stéride est obtenu à partir de lanoline.

15. Procédé de traitement selon l'une quelconque des revendications 1 à 14, **caractérisé en ce que** ladite composition cosmétique comprend en outre un composé choisi parmi les silicones sous forme soluble, dispersée, micro ou nano dispersée, volatiles ou non volatiles, les agents épaississants, les agents tensioactifs non ioniques, anioniques, cationiques et amphotères, les agents conditionneurs, les agents adoucissants, les agents anti-mousse, les agents hydratants, les agents émollients, les plastifiants, les filtres solaires hydrosolubles et liposolubles, siliconés ou non siliconés, les colorants permanents ou temporaires, les pigments minéraux ou organiques, colorés ou non colorés, les charges minérales, les argiles, les nacres ou agents, les opacifiants, les colloïdaux, les parfums, les peptisants, les conservateurs, les céramides, et pseudo-céramides, les vitamines et les provitamines dont le panthénol, les protéines, les agents séquestrants, les agents solubilisants, les agents alcanisants, les agents anti-corrosion, les corps gras autres que les cérides et les stérides, tels que les huiles végétales, animales, minérales et synthétiques, et leurs mélanges.

16. Procédé de traitement selon l'une quelconque des revendications 1 à 15, **caractérisé en ce que** ladite composition cosmétique est une composition liquide comprenant un solvant physiologiquement acceptable constitué par de l'eau ou par un ou plusieurs solvants organiques, ou encore par un mélange d'eau et d'un ou plusieurs solvants organiques.

17. Procédé de traitement selon la revendication 16, **caractérisé en ce que** le solvant organique est choisi dans le groupe comprenant l'eau, les alcools inférieurs en C1 à C4 tels que l'éthanol, l'isopropanol, l'acétone, la méthylethylcétone, l'acétate d'éthyle, l'acétate de méthyle, l'acétate de butyle, le diethoxyéthane, le diméthoxyéthane, les alcanes C6 à C10 et leurs mélanges.

18. Procédé de traitement selon l'une quelconque des revendications 1 à 17, **caractérisé en ce que** la composition cosmétique comprend, par rapport au poids total de la composition, 0,1% à 30% en poids de polymère fixant non cristallin ou semi-cristallin et/ou de stéride, et de préférence 0,5% à 20% en poids de polymère fixant non cristallin ou semi-cristallin et/ou de stéride.

19. Procédé de traitement selon l'une quelconque des revendications 1 à 18, **caractérisé en ce que** la composition cosmétique comprend, par rapport au poids total de la composition, 0,0001% à 40% en poids d'adjuvant cosmétique, et de préférence 0,001% à 20% en poids d'adjuvant cosmétique.

20. Utilisation du procédé selon l'une quelconque des revendications 1 à 19 pour modifier de manière durable la forme de la chevelure.

## Patentansprüche

1. Verfahren zur Behandlung von Haarfasern, wodurch in die Kopfbehaarung alle Arten von Motiven und Formen geprägt werden können, **dadurch gekennzeichnet, dass** es mindestens die nachstehenden aufeinanderfolgenden Schritte umfasst:
- Aufbringen eines Reduktionsmittels auf die Haarfasern,
- Spülen der Haarfasern,
- Aufbringen einer kosmetischen Zusammensetzung auf die Haarfasern, die mindestens einen Sterolester und/oder mindestens ein nicht-kristallines oder semi-kristallines fixierendes Polymer umfasst,
- Erhöhung der Temperatur der Haarfasern mithilfe einer Wärmequelle auf eine Temperatur von 50°C bis 280°C, wobei die Wärmequelle ein Lockenstab, ein Glätteisen oder ein Welleneisen ist,
- Aufbringen eines Oxidationsmittels auf die Haarfasern.

2. Verfahren zur Behandlung nach Anspruch 1, **dadurch gekennzeichnet, dass** das nicht-kristalline oder semikristalline fixierende Polymer aus anionischen, kationischen, amphoteren oder nicht-ionischen fixierenden Polymeren ausgewählt wird.

3. Verfahren zur Behandlung nach einem der Ansprüche 1 und 2, **dadurch gekennzeichnet, dass** das Eisen aus einer Klemme besteht, die zwei Arme umfasst, wobei der Querschnitt jedes Arms rechteckig, gekrümmt, dreieckig oder polyhexagonal geformt ist.

4. Verfahren zur Behandlung nach Anspruch 3, **dadurch gekennzeichnet, dass** die Arme der Klemme einen symmetrischen oder zueinander komplementären Querschnitt besitzen.

5. Verfahren zur Behandlung nach einem der Ansprüche 1 und 2, **dadurch gekennzeichnet, dass** das Eisen aus einem einzigen Heizelement besteht, um das die Haarfasern herumgewickelt werden.

6. Verfahren zur Behandlung nach Anspruch 5, **dadurch gekennzeichnet, dass** das Element einen Querschnitt besitzt, dessen Form aus einer runden, ovalen, quadratischen, rechteckigen, quaderförmigen, trapezoiden, pentagonalen, hexagonalen, oktogonalen und triangulären Form ausgewählt wird.

7. Verfahren zur Behandlung nach einem der Ansprüche 1 bis 6, **dadurch gekennzeichnet, dass** der Schritt des Aufbringens einer kosmetischen Zusammensetzung auf die Haarfasern, die mindestens ein nicht-kristallines oder semi-kristallines fixierendes Polymer und/oder mindestens einen Sterolester umfasst, und der Schritt der Erhöhung der Temperatur der Haarfasern gleichzeitig erfolgen.

8. Verfahren zur Behandlung nach einem der Ansprüche 1 bis 7, **dadurch gekennzeichnet, dass** das Reduktionsmittel ausgewählt wird aus Thiolen, wie Thioglykolsäure, Thiomilchsäure, 3-Mercaptopropionsäure, Thioäpfelsäure, 2,3-Dimercaptobernsteinsäure, Cystein, N-Glycyl-L-cystein, L-Cysteinylglycin sowie deren Estern und Salzen, Thioglycerin, Cysteamin und dessen C₁-C₄-Acyl-Derivaten, N-Mesylcysteamin, N-Acetylcystein, N-Mercaptoalkylamiden von Zuckern, wie N-(Mercapto-2-ethyl)gluconamid, Pantethein, N- (Mercaptoalkyl) - ω)-hydroxyalkylamiden, N-Mono- oder N,N-Dialkylmercapto-4-butyramiden, Aminomercaptoalkylamiden, Derivaten von N-(Mercaptoalkyl)succinaminsäuren und N-(Mercaptoalkyl)-succinamiden, Alkylaminomercaptoalkylamiden, dem azeotropen Gemisch von 2-Hydroxypropylthioglykolat und (2-Hydroxy-1-methyl)ethylthioglykolat, Mercaptoalkylaminoamiden, N-Mercaptoalkylalkandiamiden und deren Gemischen.

9. Verfahren zur Behandlung nach einem der Ansprüche 1 bis 8, **dadurch gekennzeichnet, dass** das Oxidationsmittel ausgewählt wird aus Wasserstoffperoxid, Wasserstoffperoxid, Harnstoffperoxid, alkalischen Metallbromaten, Persalzen, vorzugsweise Perboraten und Persulfaten, Enzymen, vorzugsweise Peroxidasen und 2 Elektronen übertragenden Oxidoreduktasen.

10. Verfahren zur Behandlung nach einem der Ansprüche 1 bis 9, **dadurch gekennzeichnet, dass** die kosmetische Zusammensetzung außerdem mindestens ein Wachs umfasst.

11. Verfahren nach Anspruch 10, wobei das Wachs ein Monoester von C14- bis C30-Fettsäuren und gesättigten und unverzweigten primären C16- bis C36-Alkoholen ist.

12. Verfahren zur Behandlung nach einem der Ansprüche 1 bis 11, **dadurch gekennzeichnet, dass** das Wachs ausgehend von Carnauba-, Candelilla- oder Bienenwachs erhalten wird.

13. Verfahren zur Behandlung nach einem der Ansprüche 1 bis 12, **dadurch gekennzeichnet, dass** der Sterolester ein Ester aus C14- bis C30-Fettsäuren und Sterolen ist.

14. Verfahren zur Behandlung nach einem der Ansprüche 1 bis 13, **dadurch gekennzeichnet, dass** der Sterolester ausgehend von Lanolin erhalten wird.

15. Verfahren zur Behandlung nach einem der Ansprüche 1 bis 14, **dadurch gekennzeichnet, dass** die kosmetische Zusammensetzung zudem eine Verbindung umfasst, die ausgewählt wird aus flüchtigen oder nicht-flüchtigen Silikonen in flüssiger, dispergierter, mikro- oder nanodispergierter Form, Verdickungsmitteln, nicht-ionischen, anionischen, kationischen und amphoteren oberflächenaktiven Mitteln, Konditionierungsmitteln, Weichspülern, Antischaummitteln, Hydratisierungsmitteln, Hautpflegemitteln, Weichmachern, wasserlöslichen und lipidlöslichen silikonhaltigen oder nichtsilikonhaltigen Sonnenschutzfiltern, permanenten oder temporären Farbstoffen, mineralischen oder organischen gefärbten oder ungefärbten Pigmenten, mineralischen Füllstoffen, Tonen, Perlmuttern oder Mitteln, Trübungsmitteln, Kolloiden, Parfums, Peptisierungsmitteln, Konservierungsmitteln, Ceramiden, Pseudoceramiden, Vitaminen und Provitaminen, darunter Panthenol, Proteinen, Sequestrierungsmitteln, Solubilisierungsmitteln, Alkanisierungsmitteln, Korrosionsschutzmitteln, anderen Fettkörpern als Wachse und Sterolester, wie pflanzliche, tierische, mineralische und synthetische Öle, und deren Gemischen.

16. Verfahren zur Behandlung nach einem der Ansprüche 1 bis 15, **dadurch gekennzeichnet, dass** die kosmetische Zusammensetzung eine flüssige Zusammensetzung ist, die ein physiologisch annehmbares Lösungsmittel umfasst, das aus Wasser oder aus einem oder mehreren organischen Lösungsmitteln oder auch aus einem Gemisch von Wasser und einem oder mehreren organischen Lösungsmitteln besteht.

17. Verfahren zur Behandlung nach Anspruch 16, **dadurch gekennzeichnet, dass** das organische Lösungsmittel aus der Gruppe ausgewählt wird, die Wasser, niedere C1- bis C4-Alkohole, wie Ethanol, Isopropanol, Aceton, Methylethylketon, Essigsäureethylester, Essigsäuremethylester, Essigsäurebutylester, Diethoxyethan, Dimethoxyethan, C6- bis C10-Alkane und deren Gemische umfasst.

18. Verfahren zur Behandlung nach einem der Ansprüche 1 bis 17, **dadurch gekennzeichnet, dass** die kosmetische Zusammensetzung, bezogen auf das Gesamtgewicht der Zusammensetzung, 0,1 Gew.-% bis 30 Gew.-% nicht-kristallines oder semi-kristallines fixierendes Polymer und/oder Sterolester und vorzugsweise 0,5 Gew.-% bis 20 Gew.-% nicht-kristallines oder semi-kristallines fixierendes Polymer und/oder Sterolester umfasst.

19. Verfahren zur Behandlung nach einem der Ansprüche 1 bis 18, **dadurch gekennzeichnet, dass** die kosmetische Zusammensetzung, bezogen auf das Gesamtgewicht der Zusammensetzung, 0,0001 Gew.-% bis 40 Gew.-% eines kosmetischen Hilfsstoffs und vorzugsweise 0,001 Gew.-% bis 20 Gew.-% eines kosmetischen Hilfsstoffs umfasst.

20. Verwendung des Verfahrens nach einem der Ansprüche 1 bis 19, um die Form der Kopfbehaarung dauerhaft zu modifizieren.

## Claims

1. Method for treating hair fibres that makes it possible to impart to the hair patterns and shapes of any type, **characterized in that** it comprises at least the following successive steps:
- applying a reducing agent to the hair fibres,
- rinsing the hair fibres,
- applying to the hair fibres a cosmetic composition comprising at least one sterol ester and/or at least one non-crystalline or semi-crystalline fixing polymer,
- increasing the temperature of the hair fibres by means of a heat source, to a temperature ranging from 50°C to 280°C, said heat source being a curling iron, a straightening iron or a crimping iron,
- applying an oxidizing agent to the hair fibres.

2. Treatment method according to Claim 1, **characterized in that** the non-crystalline or semi-crystalline fixing polymer is chosen from anionic, cationic, amphoteric or non-ionic fixing polymers.

3. Treatment method according to either one of Claims 1 and 2, **characterized in that** the iron consists of tongs comprising two arms, the cross section of each arm being of rectangular, curved, triangular or polyhexagonal shape.

4. Treatment method according to Claim 3, **characterized in that** the arms of the tongs have a cross section that is symmetrical or complementary to one another.

5. Treatment method according to either one of Claims 1 and 2, **characterized in that** the iron is composed of a single heating element around which the hair fibres are wound.

6. Treatment method according to Claim 5, **characterized in that** said element has a cross section, the shape of which is chosen from a circular, oval, square, rectangular, parallelepipedal, trapezoidal, pentagonal, hexagonal, octagonal and triangular shape.

7. Treatment method according to any one of Claims 1 to 6, **characterized in that** the step of applying to the hair fibres a cosmetic composition comprising at least one non-crystalline or semi-crystalline fixing polymer, and/or at least one sterol ester and the step of increasing the temperature of the hair fibres are simultaneous.

8. Treatment method according to any one of Claims 1 to 7, **characterized in that** the reducing agent is chosen from thiols such as thioglycolic acid, thiolactic acid, 3-mercaptopropionic acid, thiomalic acid, 2,3-dimercaptosuccinic acid, cysteine, N-glycyl-L-cysteine, L-cysteinylglycine and also the esters and salts thereof, thioglycerol, cysteamine and the C₁-C₄ acylated derivatives thereof, N-mesylcysteamine, N-acetylcysteine, N-mercaptoalkylamides of sugars such as N-(2-mercaptoethyl)gluconamide, pantetheine, N-(mercaptoalkyl)-ω-hydroxyalkylamides, N-mono- or N,N-dialkylmercapto-4-butyramides, aminomercaptoalkylamides, derivatives of N-(mercaptoalkyl)succinamic acids and of N-(mercaptoalkyl)succinimides, alkylaminomercaptoalkylamides, the azeotropic mixture of 2-hydroxypropyl thioglycolate and (2-hydroxy-1-methyl)ethyl thioglycolate, mercaptoalkylaminoamides, N-mercaptoalkylalkanediamides, and mixtures thereof.

9. Treatment method according to any one of Claims 1 to 8, **characterized in that** the oxidizing agent is chosen from hydrogen peroxide, aqueous hydrogen peroxide solution, urea peroxide, alkali metal bromates, persalts, preferably perborates and persulfates, enzymes, preferably peroxydases and two-electron oxidoreductases.

10. Treatment method according to any one of Claims 1 to 9, **characterized in that** the cosmetic composition also comprises at least one ceride.

11. Method according to Claim 10, said ceride being a monoester of C₁₄ to C₃₀ fatty acids and of C₁₆ to C₃₆ saturated and unbranched primary alcohols.

12. Treatment method according to any one of Claims 1 to 11, **characterized in that** the ceride is obtained from carnauba wax, candelilla wax or beeswax.

13. Treatment method according to any one of Claims 1 to 12, **characterized in that** the sterol ester is an ester of C₁₄ to C₃₀ fatty acids and of sterols.

14. Treatment method according to any one of Claims 1 to 13, **characterized in that** the sterol ester is obtained from lanolin.

15. Treatment method according to any one of Claims 1 to 14, **characterized in that** said cosmetic composition also comprises a compound chosen from volatile or nonvolatile silicones in soluble, dispersed, microdispersed or nanodispersed form, thickeners, non-ionic, anionic, cationic and amphoteric surfactants, conditioners, softeners, anti-foaming agents, moisturizers, emollients, plasticizers, silicone or non-silicone water-soluble and liposoluble sunscreens, permanent or temporary dyes, coloured or colourless, mineral or organic pigments, mineral fillers, clays, nacres or agents, opacifiers, colloids, fragrances, peptizers, preservatives, ceramides and pseudo-ceramides, vitamins and provitamins including panthenol, proteins, sequestrants, solubilizing agents, alkalinizing agents, anti-corrosion agents, fatty substances other than cerides and sterol esters, such as plant, animal, mineral and synthetic oils, and mixtures thereof.

16. Treatment method according to any one of Claims 1 to 15, **characterized in that** said cosmetic composition is a liquid composition comprising a physiologically acceptable solvent consisting of water or of one or more organic solvents, or else of a mixture of water and of one or more organic solvents.

17. Treatment method according to Claim 16, **characterized in that** the organic solvent is chosen from the group comprising water, C₁ to C₄ lower alcohols such as ethanol, isopropanol, acetone, methyl ethyl ketone, ethyl acetate, methyl acetate, butyl acetate, diethoxyethane, dimethoxyethane, C₆ to C₁₀ alkanes and mixtures thereof.

18. Treatment method according to any one of Claims 1 to 17, **characterized in that** the cosmetic composition comprises, with respect to the total weight of the composition, 0.1% to 30% by weight of non-crystalline or semi-crystalline fixing polymer and/or of sterol ester, and preferably 0.5% to 20% by weight of non-crystalline or semi-crystalline fixing polymer and/or sterol ester.

19. Treatment method according to any one of Claims 1 to 18, **characterized in that** the cosmetic composition comprises, with respect to the total weight of the composition, 0.0001% to 40% by weight of cosmetic adjuvant, and preferably 0.001% to 20% by weight of cosmetic adjuvant.

20. Use of the method according to any one of Claims 1 to 19, for lastingly modifying the shape of the hair.
